# EUROPEAN PATENT SPECIFICATION

(11) **EP 2 262 480 B1**
(45) Date of publication and mention of the grant of the patent: **14.02.2018**
(21) Application number: 09717401.5
(22) Date of filing: 04.03.2009
(51) Int. Cl.: A61K 39/385

(54) **IMMUNOMODULATOR PARTICLES**
IMMUNOMODULATOR-TEILCHEN
PARTICULES IMMUNOMODULATRICES

(30) Priority: 04.03.2008 US 68227 P
(43) Date of publication of application: 22.12.2010
(73) Proprietor: Liquidia Technologies, Inc., Research Triangle Park, NC 27709 (US)
(72) Inventor: HUBBY, Bolyn, Cary NC 27511 (US); MURPHY, Andrew, Cary NC 27519 (US); KINDIG, Jeff, Durham NC 27713 (US); WHITE, Jesse, Chapel Hill NC 27514 (US); ROTH, Samantha, Morrisville NC 27560 (US); GALLOWAY, Ashley, Cary NC 27519 (US); COPP, Laura, Raleigh NC 27614 (US)
(74) Representative: Vossius & Partner Patentanwälte Rechtsanwälte mbB
(86) International application number: PCT/US2009/036068
(87) International publication number: WO 2009/111588

(56) References cited:
- WO-A2-2006/083874
- WO-A2-2007/024323
- WO-A2-2008/045486
- US-A1- 2004 009 943
- US-A1- 2007 275 007
- KREUTER J ET AL: "Influence of the particle size on the adjuvant effect of particulate polymeric adjuvants", VACCINE, ELSEVIER LTD, GB, vol. 4, no. 2, 1 June 1986 (1986-06-01), pages 125-129, XP023710253, ISSN: 0264-410X, DOI: 10.1016/0264-410X(86)90051-4 [retrieved on 1986-06-01]
- KANCHAN ET AL: "Interactions of antigen-loaded polylactide particles with macrophages and their correlation with the immune response", BIOMATERIALS, ELSEVIER SCIENCE PUBLISHERS BV., BARKING, GB, vol. 28, no. 35, 9 October 2007 (2007-10-09), pages 5344-5357, XP022290574, ISSN: 0142-9612, DOI: 10.1016/J.BIOMATERIALS.2007.08.015
- SALEM A K ET AL: "Multi-component nanorods for vaccination applications; Multi-component nanorods for vaccination applications", NANOTECHNOLOGY, IOP, BRISTOL, GB, vol. 16, no. 4, 1 April 2005 (2005-04-01), pages 484-487, XP020091001, ISSN: 0957-4484, DOI: 10.1088/0957-4484/16/4/025
- J. A. CHAMPION: "From the Cover: Role of target geometry in phagocytosis", PROCEEDINGS OF THE NATIONAL ACADEMY OF SCIENCES, vol. 103, no. 13, 28 March 2006 (2006-03-28), pages 4930-4934, XP55018644, ISSN: 0027-8424, DOI: 10.1073/pnas.0600997103
- GRATTON ET AL: "Nanofabricated particles for engineered drug therapies: A preliminary biodistribution study of PRINT<(>TM) nanoparticles", JOURNAL OF CONTROLLED RELEASE, ELSEVIER, AMSTERDAM, NL, vol. 121, no. 1-2, 31 July 2007 (2007-07-31), pages 10-18, XP022179902, ISSN: 0168-3659, DOI: 10.1016/J.JCONREL.2007.05.027
- DAVIDE PANTAROTTO ET AL: "Immunization with Peptide-Functionalized Carbon Nanotubes Enhances Virus-Specific Neutralizing Antibody Responses", CHEMISTRY & BIOLOGY, vol. 10, no. 10, 1 October 2003 (2003-10-01), pages 961-966, XP55018645, ISSN: 1074-5521, DOI: 10.1016/j.chembiol.2003.09.011
- WEN JIANG ET AL: "Nanoparticle-mediated cellular response is size-dependent", NATURE NANOTECHNOLOGY, vol. 3, no. 3, 1 March 2008 (2008-03-01), pages 145-150, XP55018462, ISSN: 1748-3387, DOI: 10.1038/nnano.2008.30
- YIN WIN K ET AL: "Effects of particle size and surface coating on cellular uptake of polymeric nanoparticles for oral delivery of anticancer drugs", BIOMATERIALS, ELSEVIER SCIENCE PUBLISHERS BV., BARKING, GB, vol. 26, no. 15, 1 May 2005 (2005-05-01), pages 2713-2722, XP027768110, ISSN: 0142-9612 [retrieved on 2005-05-01]
- COOMBES ET AL.: 'Resorbable lamellar particles of polylactide as adjuvants for influenza virus vaccines' BIOMATERIALS vol. 19, 1998, pages 1073 - 1081, XP004161369
- VENKATAPRASAD N ET AL: "Induction of cellular immunity to a mycobacterial antigen adsorbed on lamellar particles of lactide polymers", VACCINE, ELSEVIER LTD, GB, vol. 17, no. 15-16, 9 April 1999 (1999-04-09), pages 1814-1819, XP004165027, ISSN: 0264-410X, DOI: 10.1016/S0264-410X(98)00372-7
- L. Garcia-Contreras ET AL: "Immunization by a bacterial aerosol", Proceedings of the National Academy of Sciences, vol. 105, no. 12, 25 March 2008 (2008-03-25), pages 4656-4660, XP055055946, ISSN: 0027-8424, DOI: 10.1073/pnas.0800043105
- "Novel Technology May Pave Way for Next Generation Vaccines", EON, 28 April 2009 (2009-04-28), XP055228352, Retrieved from the Internet: URL:Novel Technology May Pave Way for Next Generation Vaccines [retrieved on 2015-11-13]

## Description

### TECHNICAL FIELD OF THE INVENTION

The present invention relates, in some embodiments, to synthetic vaccine nanoparticles formed from a rapid, cell-free manufacturing process. More particularly, in some embodiments the nanoparticles include an antigen and/or an immuno enhancement agent.

### BACKGROUND OF THE FIELD OF THE INVENTION

For many infectious diseases, including malaria, tuberculosis, anthrax, tularemia, brucellosis, Hepatitis C infections, histoplasmosis, coccidioidomycosis, viral hemorrhagic fevers, bubonic plaque, viral encephalitis, Yellow Fever, and viral and bacterial gastroenteritis, there remains no available or effective vaccine. New vaccines are needed to address these diseases. In addition, challenges in antigen variability and type of immunity require novel approaches.

In any composition suitable for use as a vaccine, it is essential that the conformational integrity and immunogenic epitopes and antigenic sites be preserved intact. Changes in the structural configuration, chemical charge, or spatial orientation of these molecules and compounds may result in partial or total loss of antigenic activity and utility. The ability of an associated carrier particle to have minimal undesirable reactions in the vaccine and yet facilitate interaction of the antigenic compound with the immune system are primary concerns. All of these factors must be taken into account when preparing a composition as a conjugate that is to be used as a vaccine or as biomaterial for recognition of specific receptors.

To induce strong immune responses to a given antigen; proteins, adjuvants or other immuno enhancement agents are needed. To get an optimum immune response the antigen and agent(s) often must be co-delivered. Techniques for co-delivery have included fusing the two proteins, however, these techniques include drawbacks including physical interference between the two components, uncontrolled presentation of each component to the immune system, limitations on fusion proteins and compositions available to this technique, among other drawbacks.
Previously, nanoparticle fabrication methods, systems and materials have been described wherein the nanoparticles are molded in nano-scale molds fabricated from non-wetting, low surface energy polymeric materials (WO 2007/024323 A2). Moreover, particle compositions for controlled delivery of nucleic acids have been described wherein the micro- and nano-scale molds are fabricated from non-wetting, low surface energy polymeric materials (WO2008/045486).

Other techniques include the use of compositions that contain alum, traditional bilayer or multilamellar liposomes, polymeric particles, and virus-like particles. Such techniques have significant drawbacks, including physical and chemical stability, compatibility with a broad range or antigens, inability to codeliver antigens and immunomodulators, and safety concerns due to cell or egg-based production and the risk for recombinant virus. Therefore, new vaccines are needed that provide both antibody and cellular responses, are compatible with broad range of antigens, are capable of co-delivery and multi-valency, and have a safe and reproducible manufacturing process to ensure a consistent product.

### SUMMARY OF THE INVENTION

The present invention relates to the embodiments as characterized in the claims. Thus, it relates to the following items.
1. A vaccine composition for administration to a subject comprising:
   a plurality of particles, wherein each particle:
      is a nanometer-sized particle,
      is coupled with an antigen, and
      is fabricated with an aspect ratio of from 2:1 to 4:1,
      wherein the vaccine composition is engineered to trigger immunogenicity when administered to the subject, wherein the immunogenicity decreases with increased aspect ratio of said particle.
2. The vaccine composition of item 1, wherein the aspect ratio is 3:1.
3. The vaccine composition of item 1 or 2, wherein the particles are further coupled with an immunostimulating agent.
4. The vaccine composition of item 3, wherein at least one of the immunostimulating agent and the antigen is coupled with the particles by a non-degradable linker, or a degradable linker.
5. The vaccine composition of item 3 or 4, wherein the immunostimulating agent is physically separated from the antigen.
6. The vaccine composition of any of item 3 to 5, wherein the particles are configured to time release different doses of the antigen and the immunostimulating agent.
7. The vaccine composition of item 1 or 2, wherein the particles are configured to time release different doses of the antigen.
8. The vaccine composition of any of item 1 to 7, wherein the particles are configured to degrade at predetermined rates and/or in response to predetermined environmental conditions.
9. The vaccine composition of any of item 1 to 8, wherein the particles are configured to provide both priming and boosting capability in a dose of the vaccine composition.
10. A vaccine composition of item 1 wherein:
   the plurality of particles are substantially equivalently sized and shaped and comprise a PLGA composition; and
   the antigen, preferably HA protein, is adsorbed on a surface of the particles.
11. The vaccine composition of item 10, wherein substantially each particle of the plurality of particles has a first dimension of 80 nanometers.
12. The vaccine composition of item 1, wherein the composition comprises biodegradable materials.

There is also disclosed a method of stimulating an immune response in a subject includes administering to the subject a plurality of particles, wherein each particle is coupled with an immuno stimulating agent and a protein. It is disclosed that, the administration of the plurality of particles may result in an antibody titer at least about 10 times greater than that caused by administration of the immuno stimulating agent and the protein not coupled with the particles. It is disclosed that substantially each particle of the plurality of particles may comprise a particle having dimensions of about 200 nm by about 200 nm by about 200 nm. It is disclosed that substantially each particle of the plurality of particles may comprise a particle having dimensions of about 2 µm by about 2 µm by about 2 µm, and wherein the administration of the plurality of particles results in an antibody titer at least about 100 times greater than that caused by administration of the immuno stimulating agent and the protein not coupled with the particles.

There is also disclosed a method of stimulating an immune response in a subject includes administering a micrometer-sized particle coupled with an antigen to the subject, wherein increasing the aspect ratio of the micrometer-sized particle increases the immune response. It is disclosed that the aspect ratio of the micrometer-sized particle may be about 3:1 and the immune response is about 2 to about 3 times greater than that caused by administration of a particle having an aspect ratio of about 1:1. It is disclosed that the micrometer-sized particle may include a particle having dimensions of about 1 micrometer by about 1 micrometer by about 3 micrometers. It is disclosed that the micrometer-sized particle comprises a particle having dimensions of about 2 micrometers by about 2 micrometers by about 6 micrometers. It is disclosed that the aspect ratio of the micrometer-sized particle is about 10:1 and the immune response is about 3 to about 4 times greater than that caused by administration of a particle having an aspect ratio of about 1:1. It is disclosed that the micrometer-sized partilce may comprise a particle having dimensions of about 1 micrometer by about 1 micrometers by about 10 micrometers.

There is also disclosed a method of decreasing an immune response caused by an antigen coupled to a nanometer-sized particle includes increasing the aspect ratio of the nanometer-sized particle.

There is also disclosed a method of substantially inhibiting an immune response caused by an immuno stimulating agent administered to a subject includes coupling the immuno stimulating agent to a particle through a non-degradable linker prior to administering the immuno stimulating agent to the subject. It is disclosed that the method may result in a substantially inhibited immune response following an initial administration of the immuno stimulating agent to the subject. It is disclosed that the method may result in a substantially inhibited immune response following a booster administration of the immuno stimulating agent to the subject.

A vaccine particle composition as disclosed herein includes a plurality of particles configured to release a first protein at a first rate and a second protein at a second rate and configured to provide priming and boosting capability in a single dose.

### BRIEF DESCRIPTION OF THE FIGURES

FIG. 1 shows response at 3 weeks following an initial injection of vaccine particles according to one embodiment of the present invention where antibody levels are 1,280 for 200 nm vaccine particles; 10,240 for 2 micrometer vaccine particles; and 101 for rProtein;
FIG. 2 shows another response following an initial injection of vaccine particles according to one embodiment of the present invention;
FIG. 3 shows yet a further response following an initial injection of vaccine particles according to one embodiment of the present invention;
FIG. 4 shows a response to vaccine particles according to one embodiment of the present invention following initial injection comparing non-degradable and degradable linkers;
FIG. 5 shows a response to vaccine particles according to one embodiment of the present invention following boost injection comparing non-degradable and degradable linkers;
FIG. 6 shows 80 nm x 80 nm x 360 nm poly(dimethyaminomethacrylate) (PLGA) vaccine particles according to one embodiment of the present invention;
FIG. 7 shows a schematic of biotin-avidin linkage system for vaccine particles according to one embodiment of the present invention; and
FIG. 8 shows a hemagglutination assay, according to one embodiment of the present invention, showing a composite image comparing (1) 200 nm x 200 nm x 200 nm vaccine particles coated with mouse serum albumin (Control); (2) 200 nm x 200 nm x 200 nm vaccine particles coated with HA protein (Active); (3) HA protein positive control; and (4) negative control, where hemagglutination is shown in the outlined wells indicating the presence of HA protein on the surface of the particles.

### DETAILED DESCRIPTION OF EMBODIMENTS OF THE INVENTION

Some embodiments of the present invention generate vaccines through nanoparticle molding techniques. One surprising result is that combining antigens, for example influenza HA ("HA") (Influenza Wyoming HA protein from Protein Sciences Corporation), with an immuno stimulatory or enhancement agent, for example flagellin or IL-12 (eBiosciences, Inc.), on or in a nanoparticle can trigger a robust adaptive immune response. Moreover, the antigens and/or the immuno enhancement agent (hereinafter referred to as "agent") can be packaged within or presented on the exterior of the nanoparticle, as may be optimized for particular applications and/or combinations of antigen/agent. Furthermore, the ratios of immuno enhancement agents to antigen component of the nanoparticle can be configured for and tailored for particular applications. :
One or more immuno enhancement agents can be included with the nanoparticle and one or more antigens. The nanoparticle can also include cell or tissue specific binding or targeting agents.

The PRINT® technology (Liquidia Technologies, North Carolina) is a nanoparticle manufacturing process that uniquely allows control over particle size, shape, composition, surface functionality, and other physical characteristics such as modulus and the like. Use of this technology in some embodiments of the present invention allows for unique co-delivery strategies, shape and size-specific immunogenicity, viral-free delivery of biological cargo such as antigen and immune enhancement agents, synthetic delivery vectors and multiple delivery routes, including parental, nasal, oral, subcutaneous, intradermal, intramuscular, intraperitoneal, inhaled, and the like.

The composition of the nanoparticles as disclosed herein can be configured to time release different doses of antigen and/or antigen and agent components to optimize interaction with the immune system and antibody formation. - The composition of the nanoparticle can be configured to degrade at predetermined rates and/or in response to predetermined environmental conditions such as, for example, pH, aqueous environments, particular enzymes, or the like.
The nanoparticle can be tuned to degrade by preselecting crosslinkers and/or crosslinker density to facilitate degradation of the particle in preselected environments or at preselected degradation rates. The nanoparticle can include varied types and amounts of surface agents to screen, bind, or mask the particle to or from desired cells. The nanoparticles can also be designed, by controlling a polymer matrix of the nanoparticle, to deliver an antigen into an intracellular space before the nanoparticle is broken down, thereby providing intracellular delivery of an antigen and/or an antigen/immuno enhancement agent. Compositions as disclosed herein can include biodegradable materials, hydrophilic materials, GRAS (generally regarded as safe) materials, and the like. Particles as disclosed herein of the present invention can include or be formed of poly(ethylene glycol), poly(lactic-co-glycolic acid), polycaprolactone, and the like.

As disclosed herein, the particle composition may comprise both the antigen and the agent. As disclosed herein, the particle composition may comprise only one of the antigen or agent. One or more of the antigen or agent is conjugated to the nanoparticle surface. The ratio of agent to antibody can be controlled by controlling the composition that is molded into the nanoparticle or conjugated to the nanoparticle surface.

As disclosed herein, the amount of antigen on the surface may comprise between about 0 and about 50% of the particle by weight. The amount of antigen on the surface may comprise between about 0 and about 40% of the particle by weight.
The amount of antigen on the surface may comprise between about 0 and about 30% of the particle by weight. The amount of antigen on the surface may comprise between about 0 and about 20% of the particle by weight.
The amount of antigen on the surface may comprise between about 0.1 and about 20% of the particle by weight. The amount of antigen on the surface may comprise between about 0.1 and about 10% of the particle.

As disclosed herein, the immuno enhancement agent comprises between about 0 and about 70% of the antigen quantity. The immuno enhancement agent may comprise between about 0 and about 60% of the antigen quantity.
The immuno enhancement agent may comprise between about 0 and about 50% of i the antigen quantity. The immuno enhancement agent may comprise between about 0 and about 40% of the antigen quantity. The immuno enhancement agent may comprise between about 0 and about 30% of the antigen quantity.

The immuno enhancement agent may comprise between about 0 and about 20% of the antigen quantity. The immuno enhancement agent may comprise between about 0 and about 10% of the antigen quantity. The immuno enhancement agent may comprise between about 0.1 and about 30% of the antigen quantity. The immuno enhancement agent may comprise between about 0.1 and about 20% of the antigen quantity. The immuno enhancement agent may comprise between about 0.1 and about 10% of the antigen quantity.

As disclosed herein, the nanoparticles may provide particle mediated controlled release, which can improve response arid reduce reactogenicity to current immunomodulated vaccine technologies. The nanoparticles can also be configured to produce dose-sparing and boost sparing regimes as well as increase product stability and shelf-life, for example, by manipulating matrix compositions. Nanoparticle packaging of the immunomodulated vaccines can further minimize breakdown *in vivo* or control *in vivo* breakdown to preselected environmental conditions. The nanoparticles may further provide for potential co-packaging of additional immunomodulators and/or antigens within a single nanoparticle or within a plurality of nanoparticles that comprise a single or multiple dose(s).

As disclosed herein , the nanoparticles may provide for multivalent vaccines and Th1/Th2 modulation. The nanoparticles may also produce particle mediated delivery to cytosol via endosomal escape, allowing efficient presentation of antigens for T cell induction. Nanoparticles as disclosed herein can also provide for co-packaging of flagellin and immunogen within or on a single particle that can avoid requirement for fusion protein. The present nanoparticles may enhance maintenance of correct conformation of flagellin and/or immunogen, increase APC targeting, lower dose requirement, decreased reactogenicity, combinations thereof, and the like. As disclosed herein, the correct conformation of the flagellin and/or immunogen may be enhanced on the nanoparticles of the present invention because the immunogen and/or flagellin are attached to the particle after the particle is fabricated and using all aqueous conditions and chemistries.

Nanoparticle fabrication described in the nanoparticle molding technique (PRINT® (Liquidia Technologies, Inc. North Carolina)) allows for the facile incorporation of a number of biological agents, including antigens, immunomodulations, adjuvants, and other immuno enhancement agents within the particle matrix. The nanoparticle molding technology also allows for the attachment of targeting ligands to the surface of the particle. For vaccine applications targeting the therapeutic reduces the chances of having systemic reactogenicity and/or decreases the dosing requirements.
A particle may contain, on a surface, on the entire surface, or within the particle, one or more immuno enhancement agents and one or more antigens.
A particle may comprise one or more of the antigens of interest, an immuno enhancement agent, and a targeting agent. The nanoparticle molding technology also allows for multivalent vaccines. As disclosed herein, the nanoparticle may contain or include multiple vaccine antigens.

The nanoparticle is comprised such that there is physical separation of the antigen(s) and the immuno enhancement agent. One technique for physically separating the antigen and agent includes encapsulating the antigen and functionalizing the surface of the particle with the agent, or the inverse thereof. Another technique for physically separating the antigen and agent, includes attaching both the antigen and the agent to different areas of the same particle surface, making the particle the link between the antigen and the agent.
This allows for the shape of the proteins, i.e., conformation, to be maintained, which is important for generating appropriate immune responses, for example for antibody induction, and can be critical for the activity of immuno enhancement agents.

It is also disclosed that the particles are designed to bias the immune response towards either a Th1 or Th2 bias depending on the immuno enhancement agents that are included. The degradation of the particle matrix is designed to induce a given response. The particles may be designed to degrade in an endosome, rupturing it and creating a CD8 response. The particles may be designed to be broken down in the endosome/lysosome pathway and presented to CD4 T-cells to eventually induce more of an antibody response.

The particles may be designed to release two or more proteins at different rates. It is disclosed that each of the two or more proteins is released at different rates. It is disclosed that the release rate is tailored such that a single nanoparticle design can provide a prime and boost capability in the same dose.

Some particles may be designed to release some of the proteins quickly and other proteins bound to or combined with the nanoparticle release slowly. The cargo may be masked to increase the ability to boost later. The cargo may be protected from breakdown *in vivo* thus improving the efficacy and possibly leading to a reduced dosing requirement.

In some embodiments, the vaccine particles of the invention can be engineered with a , specific shape to elicit a desired, increased, or decreased antibody response. As disclosed herein,! in some cases high aspect ratio particle elicits a greater immunogenic response than a particle with a lower aspect ratio. In other cases, a lower aspect ratio elicits a greater immunogenic response.
Aspect ratio refers to the ratio of the longest axis to the shortest axis of a particle. The preferred particle shape may be a function of the uptake mechanism, antigen, immuno stimulatory agent, type of response, and the like. It is disclosed that particles shapes with a greater surface-to-volume ratio are preferred.

As disclosed herein particles can be fabricated with aspect ratios of at least about 1:1. Vaccine particles can be fabricated with aspect rations of at least about 2:1. Vaccine particles can be fabricated with aspect rations of at least about 3:1. Vaccine particles can be fabricated with aspect rations of at least about 4:1. Vaccine particles can be fabricated with aspect rations of at least about 5:1. Vaccine particles can be fabricated with aspect rations of at least about 6:1. Vaccine particles can be fabricated with aspect rations of at least about 7:1. Vaccine particles can be fabricated with aspect rations of at least about 8:1. Vaccine particles can be fabricated with aspect rations of at least about 9:1.
Vaccine particles can be fabricated with aspect ratios of at least about 10:1. It is also disclosed that vaccine particles can be fabricated with aspect ratios ranging from about 1:1 to about 60:1. Vaccine particles can be fabricated with aspect ratios ranging from about 1:1 to about 50:1. Vaccine particles can be fabricated with aspect ratios ranging from about 1:1 to about 40:1.
Vaccine particles can be fabricated with aspect ratios ranging from about 1:1 to about 30:1. Vaccine particles can be fabricated with aspect ratios ranging from about 1:1 to about 20:1. Vaccine particles can be fabricated with aspect ratios ranging from about 1:1 to about 15:1.
Vaccine particles can be fabricated with aspect ratios ranging from about 1:1 to about 10:1. Vaccine particles can be fabricated with aspect ratios ranging from about 1:1 to about 9:1. Vaccine particles can be fabricated with aspect ratios ranging from about 1:1 to about 8:1.
Vaccine particles can be fabricated with aspect ratios ranging from about 1:1 to about 7:1. Vaccine particles can be fabricated with aspect ratios ranging from about 1:1 to about 6:1. Vaccine particles can be fabricated with aspect ratios ranging from about 1:1 to about 5:1.
Vaccine particles can be fabricated with aspect ratios ranging from about 1:1 to about 4:1. Vaccine particles can be fabricated with aspect ratios ranging from about 1:1 to about 3:1. Vaccine particles can be fabricated with aspect ratios ranging from about 1:1 to about 2:1.
Vaccine particles can be fabricated with aspect ratios of about 1:1.
Vaccine particles can be fabricated with aspect ratios of about 2:1.
Vaccine particles can be fabricated with aspect ratios of about 3:1.
Vaccine particles can be fabricated with aspect ratios of about 4:1.
Vaccine particles can be fabricated with aspect ratios of about 5:1.
Vaccine particles can be fabricated with aspect ratios of about 6:1.
Vaccine particles can be fabricated with aspect ratios of about 7:1. ,
Vaccine particles can be fabricated with aspect ratios of about 8:1.
Vaccine particles can be fabricated with aspect ratios of about 9:1.
Vaccine particles can be fabricated with an aspect ratio of about 10:1.
Vaccine particles can be fabricated with an aspect ratio of about 15:1.
Vaccine particles can be fabricated with an aspect ratio of about 20:1. Vaccine particles can be fabricated with an aspect ratio of about 30:1. Vaccine particles can be fabricated with an aspect ratio of about 40:1. Vaccine particles can be fabricated with an aspect ratio of about 50:1. Vaccine particles can be fabricated with an aspect ratio of about 60:1. Vaccine particles can be fabricated with an aspect ratio of greater than about 60:1.

Accordingly, the vaccine particles as disclosed herein can be fabricated with any dimension being controlled on the nanoscale. For example, vaccine particles having the above described aspect ratios can be fabricated as particles having dimensions of about 80 nm x about 90 nm cylinder shaped particles; particles having dimensions of about 80 nm x about 80 nm x about 360 nm; particles having dimensions of about 80 nm x about 80 nm x about 2000 nm; particles having dimensions of about 80 nm x about 80 nm x about 5000 nm; particles having dimensions of about 1 micrometer x about 1 micrometer; particles having dimensions of about 1 micrometer x about 3 micrometer cylinder shaped; and particles having dimensions of about 1 micrometer x about 10 micrometer.

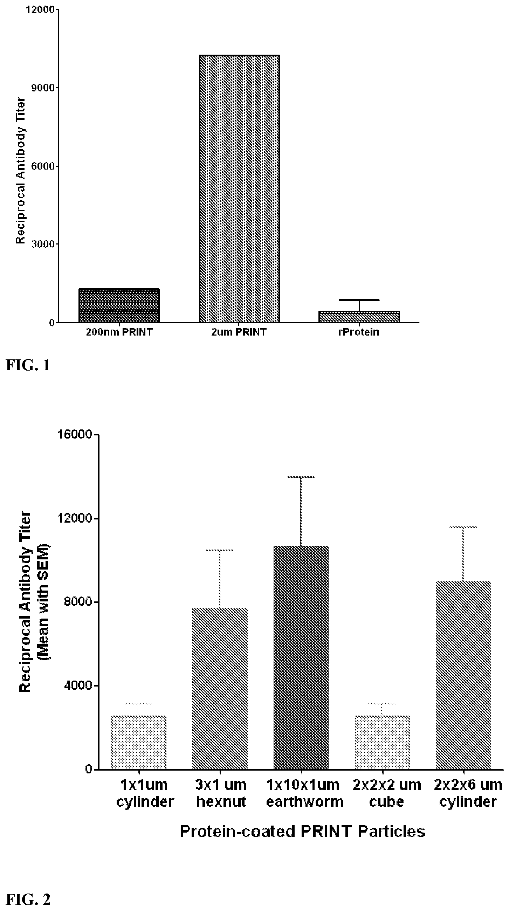

Vaccine particles as disclosed herein (for example the particles of Example 2) co-delivering HA and IL-12 induce stronger antibody responses against the HA protein as compared to the responses generated in mice that receive a mixture of two soluble proteins, as shown in FIG. 1. The increase in antibody response can range from about 10 to about 100 fold increase. Also, particle size influences the antibody response. As shown in FIG. 1, soluble protein generate a response of about 100 antibody titer, 200 nm x 200 nm x 200 nm vaccine particles as disclosed herein generate a response of about 1280 antibody titer, and 2 micrometer x 2 micrometer x 2 micrometer vaccine particles generate a response of about 10,240 antibody titer three weeks after an initial injection. The vaccine particles as disclosed herein may be tailored in shape, size, and aspect ratio to effectively co-deliver an antigen and a protein adjuvant and to obtain a desired immunogenicity.

The aspect ratio of the vaccine nanoparticles of the present invention can influence the response generated to the vaccine nanoparticle.
After initial injection, the aspect ratio of micrometer sized vaccine nanoparticles having influenza HA coupled with their surface may generate an increasing immune response as the aspect ratio of the vaccine nanoparticle increases, as shown in FIG. 2.
The immune response of the particles having an aspect ratio of about 3:1 is about 2-3 times that of the particle having an aspect ratio of 1:1. The immune response of the micrometer sized particles having an aspect ratio of about 10:1 is about 3-4 times that of the micrometer sized particle having an aspect ratio of 1:1. The antibody responses induced after a single injection are shown in FIG. 2.

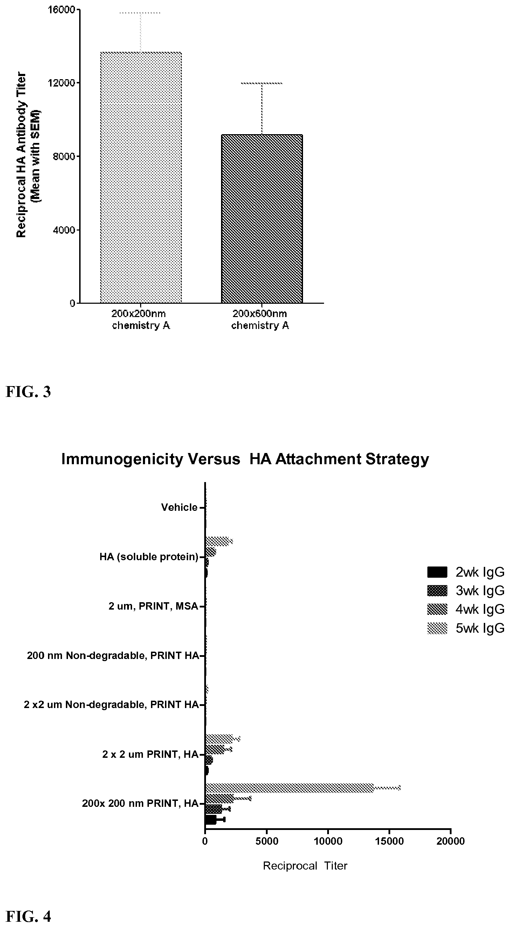

As disclosed herein the aspect ratio of nanometer sized vaccine particles may result in immune responses that decrease with increasing aspect ratios of the vaccine particles with influenza HA coupled to the surface after initial injection, as shown in FIG. 3. The composition of the vaccine particles shown in FIG. 3 includes a cross-linked polyethylene glycol (PEG) based (composed of 79% Poly(ethylene glycol) dimethacrylate, 20% aminoethyl methacrylate HCl, 1 % HCPK, and then surface treated with a biotin linker surface modification).

The vaccine particles as disclosed herein may be molded in low surface energy molds, utilizing the methods and materials described in the following patent publications: US 8, 263, 129 and US 2007/0264481; US 2011/0182805; US 2007/0264481; WO 2007/094829; and US 2009/0220789

As disclosed herein, the vaccine particles may be formulated to not create an immune response. The immunogenicity of the particle can be controlled by the conjugation of the antigen and/or the agent to the particle surface using linker group chemistry. Specifically, the immunogenicity can be controlled by the degradation kinetics of the linker groups. Higher antibody responses are found in mice injected with particles containing HA linked to the surface through a degradable disulfide crosslinker versus injection with particles containing HA linked to the surface thru a non-degradable bond. This effect can be seen following both prime and boost injections.

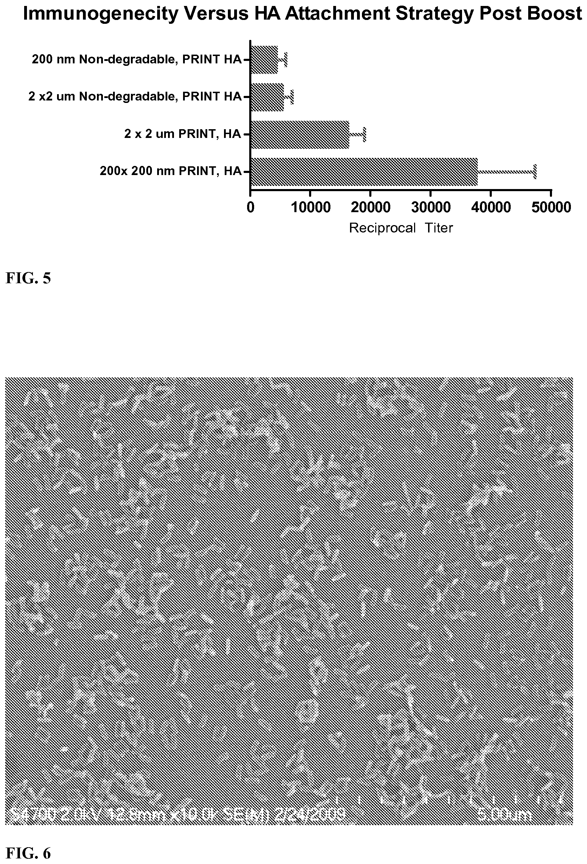

As disclosed herein, as seen in FIGS. 4 and 5, a protein linked to a nanoparticle through a non-degradable cross-linker yields no or limited activity, whereas a protein linked through a degradable cross-linker yields a response. As disclosed herein the nanoparticle has an adjuvant attached thereto through a non-degradable crosslinker such that an immune response is not generated, or minimally generated, with respect to that adjuvant. This can help maintain the adjuvant's effect over multiple injections, allow the use of highly potent adjuvants with less concern about the quantity presented, allow the use of adjuvants that often trigger adverse reactions, or the like simply by attaching the adjuvant with a non-degradable linker to the nanoparticle. As shown in FIG. 4, an anti-HA antibody response was generated in mice that received vaccine nanoparticles coated with HA protein when attached with a degradable linker but no detectable response post initial injection was generated when the HA protein was attached with a nondegradable linker. These results were seen with both 200 nm x 200 nm x 200 nm vaccine particles and 2 micrometer x 2 micrometer x 2 micrometer vaccine particles. After a second injection, as shown in FIG. 5, an anti-HA antibody response was detectable with the particles that contained the HA protein linked with the non-degradable linker, however, the response for the 200 nm vaccine particles was about 3-4 times less than the response generated with 200 nm vaccine particles having the HA attached via a degradable linker and the response for the 2 micrometer vaccine particles was about 5-6 times less than the response generated from the 2 micrometer vaccine particles having the HA attached via a degradable linker.

According to some embodiments of the present invention, vaccine particles can be formed with non-degradable linkers attaching agents, antigens, molecules or the like to which no or limited immune response is desired. For example, a targeting agent or other protein or molecule maybe attached to a vaccine particle of the present invention through a non-degradable linker and no or limited immune response will be elicited from the presence of that agent on the vaccine particle.

As disclosed herein, the antigens are delivered in a particulate form to improve immune responses to the antigen. The dose of the adjuvant and/or immuno enhanced agents may be varied to give the appropriate response.
The dose may be adjusted to give reactogenic immunomodulators sparingly while not having to reduce the amount of antigen delivered. The reactogenic proteins may be encapsulated within the particles. The particles may be targeted to cells of interest. The cells may be antigen presenting cells.
The particle may be designed to be taken up intracellularly then degraded once the particle is internalized The surface of the particle can be functionalized with one or more of antigens, immune enhancement agents, and targeting ligands. One or more of the antigens, immune enhancement agents, and targeting ligands may be attached to the surface with degradable linker groups.
One or more of the antigens, immune enhancement agents, and targeting ligands may be attached to the surface with non-degradable linker groups. One or more of the antigens, immune enhancement agents, and targeting ligands may be attached to the surface with degradable linker groups and others are attached to the surface with non-degradable linker groups. For example, a PGLA nanoparticle can have HA, IL-12, and a cell targeting ligand attached to the surface in varying quantities; the HA and IL-12 may be attached with degradable linker groups, and the cell targeting ligand may be attached via a non-degradable linker group.

Antigens used in the nanoparticle vaccines can be any type of antigen e.g., including but not limited to pathogen-related antigens, tumor-related antigens, allergy-related antigens, neural defect-related antigens, cardiovascular disease antigens, rheumatoid arthritis-related antigens, other disease-related antigens, hormones, pregnancy-related antigens, embryonic antigens and/or fetal antigens and the like. The nanoparticle vaccines may be recombinant proteins, or recombinant lipoproteins, or recombinant glycoproteins, and one or more antigens.

It is described that the nanoparticle vaccine as disclosed herein can be administered to a subject in need thereof to trigger an immune response and antibody formation against an antigen of interest. The nanoparticle vaccine can be administered by injection, inhalation, transdermal, transmucosal, anal, vaginal, ocular, ingestion, intravenous, combinations thereof, or the like. In some embodiments, the viral antigen/agent nanoparticle product, such as antigen-flagellin nanoparticle for example, can generate a response even against targets that are not very immunogenic, including highly conserved regions of viral proteins.

Upon administration into human or animal subjects, the nanoparticle vaccines can interact with, for example dendritic cells and macrophages. This interaction will have two consequences: First, the immuno stimulatory agent portion of the nanoparticle vaccine will interact with and stimulate a signaling pathway, such as the NF-.kappa.B, JNK and/or p38 pathways. Second, due to this interaction with receptors, the nanoparticle vaccine will be taken up into dendritic cells and macrophages by phagocytosis, endocytosis, or macropinocytosis, depending on the cell type, the size of the nanoparticle vaccine, and the identity of the stimulatory agent. Next, activation of the signaling pathways will lead to the induction of the expression of cytokines, chemokines, adhesion molecules, and co-stimulatory molecules by dendritic cells and macrophages and, in some cases, B-cells. Uptake of the nanoparticles will lead to the processing of the antigen(s) included in or on the nanoparticle and the presentation by the MHC class-I and MHC class-II molecules. This will generate the two signals required for the activation of naive T-cells a specific antigen signal and the co-stimulatory signal. In addition, chemokines induced by the nanoparticle vaccine will recruit naive T-cells to the APC and cytokines, like IL-12, which will induce T-cell differentiation into Th-1 effector cells. As a result, a robust T-cell immune response will be induced, which will in turn activate other aspects of the adaptive immune responses, such as activation of antigen-specific B-cells and macrophages.

Material useful can be found in US Patent numbers US7,285,535 titled Toll/interleukin-1 receptor adapter protein (TIRAP); and US6,960,343 titled Toll/interleukin-1 receptor adapter protein (TIRAP); and US Published applications US20070160623 titled Innate immune system-directed vaccines; US20070122421 titled Innate immune system-directed vaccines; US20060188933 titled IRAK-M is a negative regulator of toll-like receptor signaling; US20060130164 titled Toll/interleukin-1 receptor adapter protein (TIRAP); US20060121460 titled Toll-like receptor 11; US20050163764 titled Treatment with agonists of toll-like receptors; US20030232055 titled Innate immune system-directed vaccines; US20030224388 titled RIP2: a mediator of signaling in the innate and adaptive immune systems; US20030175287 titled Innate immune system-directed vaccines; US20030157539 titled IRAK-M is a negative regulator of toll-like receptor signaling; US20030023993 titled Toll/interleukin-1 receptor adapter protein (TIRAP); and US20020061312 titled Innate immune system-directed vaccines.
Some of the information in the above references includes immuno stimulatory proteins or agents, antigens, fusion proteins, delivery techniques, dosage regimes, and the like.

Further material useful can include US Patent number 7,060,284, discussing *inter alia* compositions comprising polypeptides and polynucleotides for stimulating the immune system and for treating malignancies associated with overexpression of the HER-2 protein; Brennan, et al., "Cowpea Mosaic Virus as a Vaccine Carrier of Heterologous Antigens," Mol. Biotechnol. 17(1):15-26 (2001), discussing *inter alia* chimeric virus particles as carriers of heterologous antigens; US Patent number 6,060,064 to Adams, et al., describing *inter alia* use of a protein carrier used to display immunogenic amino acid sequences for use as a vaccine; US Patent number 6,086,881 to Frey, et al. describing, *inter alia,* other multivalent vaccine constructs including metallic oxide particles; US Patent number 5,686,113 to Speaker, et al. describing, *inter alia,* polysaccharide-based spermine, alginate capsules, which are non-synthetic; US Patent number 6,326,021 to Schwendeman, et al. describing, *inter alia,* synthetic biocompatible base polymer of poly lactide-co-glycolide; US Patent numbers 5,709,879 to Barchfeld, et al.; 6,342,226 to Betbeder, et al.; 6,090,406 to Popescu, et al.; and Lian, et al., Trends and Developments in Liposome Drug Delivery Systems, J. of Pharma. Sci. 90(6):667-680 (2001), and van Slooten, et al., Liposomes Containing Interferon-gamma as Adjuvant in Tumor Cell Vaccines, Pharm Res. 17(1):42-48 (2000) describing, *inter alia,* nanoparticle carriers for use as vaccine made from lipids or other fatty acids; and Kreuter, "Nanoparticles and Microparticles for Drug and Vaccine Delivery," J. Anat. 189:503-505 (1996), describing, *inter alia,* non-lipid compositions.

"Vaccine" can refer to a composition comprising an antigen, and optionally other ancillary molecules, the purpose of which is to administer such compositions to a subject to stimulate an immune response specifically against the antigen and preferably to engender immunological memory that leads to mounting of an immune response should the subject encounter that antigen at some future time. Examples of other ancillary molecules are adjuvants, which are non-specific immunostimulatory molecules, and other molecules that improve the pharmacokinetic and/or pharmacodynamic properties of the antigen. Conventionally, a vaccine usually consists of the organism that causes a disease (suitably attenuated or killed) or some part of the pathogenic organism as the antigen. Attenuated organisms, such as attenuated viruses or attenuated bacteria, are manipulated so that they lose some or all of their ability to grow in their natural host. There are now a range of biotechnological approaches used to producing vaccines. (See, e.g., W. Bains (1998) Biotechnology From A to Z, Second Edition, Oxford University Press).

"Antigen" refers to a substance that is specifically recognized by the antigen receptors of the adaptive immune system. Thus, as used herein, the term "antigen" includes antigens, derivatives or portions of antigens that are immunogenic and immunogenic molecules derived from antigens. Preferably, the antigens used in the present invention are isolated antigens. Antigens that are particularly useful in the present invention include, but are not limited to, those that are pathogen-related, allergen-related, or disease-related.

An antigen is also any substance that induces a state of sensitivity and/or immune responsiveness after any latent period (normally, days to weeks in humans) and that reacts in a demonstrable way with antibodies and/or immune cells of the sensitized subject *in vivo* or *in vitro.* Examples of antigens include, but are not limited to, microbial-related antigens, especially antigens of pathogens such as viruses, fungi or bacteria, or immunogenic molecules derived from them; cellular antigens including cells containing normal transplantation antigens and/or tumor-related antigens; RR Rh antigens; antigens characteristic of, or specific to particular cells or tissues or body fluids; and allergen-related antigens such as those associated with environmental allergens (e.g., grasses, pollens, molds, dust, insects and dander), occupational allergens (e.g., latex, dander, urethanes, epoxy resins), food (e.g., shellfish, peanuts, eggs, milk products), drugs (e.g., antibiotics, anesthetics) and vaccines (e.g., flu vaccine).

Antigen processing and recognition of displayed peptides by T-lymphocytes depends in large part on the amino acid sequence of the antigen rather than the three-dimensional structure of the antigen. Thus, the antigen portion used in the nanoparticle vaccines of the present invention can contain epitopes or specific domains of interest rather than the entire sequence. In fact, the antigenic portions of the nanoparticle vaccines of the present invention can comprise one or more immunogenic portions or derivatives of the antigen rather than the entire antigen. Additionally, the nanoparticle vaccine of the present invention can contain an entire antigen with intact three-dimensional structure or a portion of the antigen that maintains a three-dimensional structure of an antigenic determinant, in order to produce an antibody response against a spatial epitope of the antigen.

Pathogen-Related Antigens. Specific examples of pathogen-related antigens include, but are not limited to, antigens selected from the group consisting of vaccinia, avipox virus, turkey influenza virus, bovine leukemia virus, feline leukemia virus, avian influenza, chicken pneumovirosis virus, canine parvovirus, equine influenza, FHV, Newcastle Disease Virus (NDV), Chicken/Pennsylvania/1/83 influenza virus, infectious bronchitis virus; Dengue virus, measles virus, Rubella virus, pseudorabies, Epstein-Barr Virus, HIV, SIV, EHV, BHV, HCMV, Hantaan, C. tetani, mumps, Morbillivirus, Herpes Simplex Virus type 1, Herpes Simplex Virus type 2, Human cytomegalovirus, Hepatitis A Virus, Hepatitis B Virus, Hepatitis C Virus, Hepatitis E Virus, Respiratory Syncytial Virus, Human Papilloma Virus, Influenza Virus, Salmonella, Neisseria, Borrelia, Chlamydia, Bordetella, and Plasmodium and Toxoplasma, Cryptococcus, Streptococcus, Staphylococcus, Haemophilus, Diptheria, Tetanus, Pertussis, Escherichia, Candida, Aspergillus, Entamoeba, Giardia, and Trypanasoma.

Cancer-Related Antigens. The methods and compositions : can also be used to produce vaccines directed against tumor-associated protein antigens such as melanoma-associated antigens, mammary cancer-associated antigens, colorectal cancer-associated antigens, prostate cancer-associated antigens and the like.

Specific examples of tumor-related or tissue-specific proteins antigens useful in such vaccines include, but are not limited to, antigens selected from the group consisting of prostate-specific antigen (PSA), prostate-specific membrane antigen (PSMA), Her-2, epidermal growth factor receptor, gp120, and p24. In order for tumors to give rise to proliferating and malignant cells, they must become vascularized. Strategies that prevent tumor vascularization have the potential for being therapeutic. The methods and compositions can also be used to produce nanoparticle vaccines directed against tumor vascularization. Examples of target antigens for such nanoparticle vaccines are vascular endothelial growth factors, vascular endothelial growth factor receptors, fibroblast growth factors and fibroblast growth factor receptors and the like.

Allergen-Related Antigens. The methods and compositions can be used to prevent or treat allergies and asthma. One or more protein allergens can be linked to one or more nanoparticle or immuno stimulating agent, producing an agent/antigen chimeric construct, and administered to subjects that are allergic to that antigen. Thus, the methods and compositions can also be used to construct nanoparticle vaccines that may suppress allergic reactions.

Specific examples of allergen-related protein antigens useful in methods and compositions include, but are not limited to: allergens derived from pollen, such as those derived from trees such as Japanese cedar (Cryptomeria, Cryptomeriajaponica), grasses (Gramineae), such as orchard-grass (Dactylis, Dactylis glomerata), weeds such as ragweed (Ambrosia, Ambrosia artemisiifolia); specific examples of pollen allergens including the Japanese cedar pollen allergens Cry j 1 (J. Allergy Clin. Immunol. (1983)71: 77-86) and Cry j 2 (FEBS Letters (1988) 239: 329-332) and the ragweed allergens Amb a I.1, Amba 1.2, Amb a 1.3, Amnb a 1.4, Amb a II etc.; allergens derived from fungi (Aspergillus, Candida, Alternaria, etc.); allergens derived from mites (allergens from Dermatophagoides pteronyssinus, Dermatophagoides farinae etc.; specific examples of mite allergens including Der p I, Der p II, Der p III, Der p VII, Der f I, Der f II, Der f III, Der f VII etc.); house dust; allergens derived from animal skin debris, feces and hair (for example, the feline allergen Fel d I); allergens derived from insects (such as scaly hair or scale of moths, butterflies, Chironomidae etc., poisons of the Vespidae, such as Vespa mandarinia); food allergens (eggs, milk, meat, seafood, beans, cereals, fruits, nuts and vegetables etc.); allergens derived from parasites (such as roundworm and nematodes, for example, Anisakis); and protein or peptide based drugs (such as insulin).

Other Disease Antigens. Also contemplated are nanoparticle vaccines directed against antigens that are associated with diseases other than cancer, allergy and asthma. As one example of many, and not by limitation, an extracellular accumulation of a protein cleavage product of .beta.-amyloid precursor protein, called "amyloid-.beta. peptide", is associated with the pathogenesis of Alzheimer's disease. (Janus et al., Nature (2000) 408: 979-982; Morgan et al., Nature (2000) 408: 982-985). Thus, the chimeric construct used in the nanoparticle vaccines can include amyloid.beta. peptide, or antigenic domains of amyloid.beta. peptide, as the antigenic portion of the construct.

"Immuno enhancement agent" or "Immuno stimulatory agent" or "agent" can mean a molecular pattern found in a microorganism but not in humans, which, can trigger an innate immune response. Thus, as used herein, the term includes any such microbial molecular pattern and is not limited to those associated with pathogenic microorganisms or microbes. As used herein, the term includes structures or derivatives thereof that are potential initiators of innate immune responses. Immunostimulatory structures can be found in, or composed of molecules including, but not limited to, lipopolysaccharides; phosphatidyl choline; cytokine adjuvants; glycans, including peptidoglycans; teichoic acids, including lipoteichoic acids; proteins, including lipoproteins and lipopeptides; outer membrane proteins (OMPs), outer surface proteins (OSPs) and other protein components of the bacterial cell walls; bacterial DNAs; single and double-stranded viral RNAs; unmethylated CpG-DNAs; mannans; mycobacterial membranes; porins; and a variety of other bacterial and fungal cell wall components, including those found in yeast.

As used herein, immuno stimulatory agents are discrete molecular structures that are shared by a large group of microorganisms. They are often conserved products of microbial metabolism, which are not subject to antigenic variability and are distinct from self-antigens, *see* Medzhitov et al. (1997) Current Opinion in Immunology 9: 4.

Immuno stimulatory agents can be composed of or found in, but are not limited to the following types of molecules: lipopolysaccharides (LPS), porins, lipid A-associated proteins (LAP), lipopolysaccharides, fimbrial proteins, unmethylated CpG motifs, bacterial DNAs, double-stranded viral RNAs, mannans, cell wall-associated proteins, heat shock proteins, glycoproteins, lipids, cell surface polysaccharides, glycans (e.g., peptidoglycans), phosphatidyl cholines, teichoic acids (e.g., lipoteichoic acids), mycobacterial cell wall components/membranes, bacterial lipoproteins (BLP), outer membrane proteins (OMP), and outer surface protein A (Osp A). Other useful Adjuvants are disclosed in Henderson et al. (1996) Microbiol. Review 60: 316; Medzhitov et al (1997) Current Opinion in Immunology 9: 4-9); The European Medicines Agency Evaluation of Medicines for Human Use, January 20, 2005, http://www.emea.europa.eu/pdfs/human/vwp/13471604en.pdf.

As disclosed herein, a preferred immuno stimulatory agent of the present invention includes those that contain a DNA-encoded protein component, such as BLP, Neisseria porin, OMP, and OspA. One immuno stimulatory agent for use is BLP because BLP is known to induce activation of the innate immune response (Henderson et al. (1996) Microbiol. Review 60: 316) and has been shown to be recognized by the immune system (Aliprantis et al. (1999) Science 285: 763).

There are also disclosed methods of screening multiple immuno enhancement agents, antigens, and/or targeting agents by fabricating a plurality of nanoparticles of the same or different compositions in large scale batch or continuous processes. The nanoparticles can include only the agent/antigen as the entire composition of the nanoparticle or the combination agent/antigen can be mixed with or encased in a third composition that can provide desired in vivo activity. Such desired in vivo activity can include, but is not limited to, increased circulation times, mask from degradation, masking from an unwanted immune response, crossing a cellular membrane, intracellular degradation, combinations thereof, or the like. Furthermore, the nanoparticles can be fabricated from the antigen, then the agent can be bound with the nanoparticle or a targeting agent can be coupled with the nanoparticle. In one screening method, large quantities of nanoparticles containing the desired antigen can be fabricated from a matrix that includes a linker group, such as a primary amine, hydroxyl group, sulfide, carboxylic acid, and the like. Immuno enhancement agents can then be chemically or physically coupled to subsets of the antigen-containing particle through these linker groups using organic chemistry known in the art. The particles can be functionalized in solution or dry powder after the particles are harvested from the mold, or functionalized directly on the array of particles in a combinatorial manner. Through this method, the particles can be functionalized to contain one or more different immunomodulators on the surface, at a controlled density, ratio, and location. This library of surface functionalized particles can then be screened in the appropriate in vitro or in vivo model to determine the optimal immune enhancement agent, combination of agents, and relative concentrations for the antigen of interest. Further screening methods include varying the concentration of the antigen within the particle in addition to the immune enhancement agents on the surface.

It is also disclosed that the nanoparticle vaccine can be used to screen a subject for the presence of an antibody or screening a series of varied stimulatory agent/antigen combination nanoparticles against a sample to determine the presence of or absence of an antibody or the function of a stimulatory agent/antigen combination. The nanoparticle can be configured with a component that, when in contact with an antibody of interest undergoes a physical or chemical change that is detectable or produces a signal, e.g., the nanoparticle can include or be a biosensor and in can be triggered when the nanoparticle binds to a specific target. Therefore, a variety of combinations of nanoparticles having different combinations and/or ratios of stimulatory agent to antigen can be administered to a subject or applied to a sample to detect the presence of an antibody or the function or viability of an agent/antigen combination.

It is also disclosed that the nanoparticles can be used to attract, bind, and facilitate flagging or filtering of particular cells or proteins from an organism or from a sample. As disclosed herein , the nanoparticles can be configured with a cell, protein or virus specific binding agent, such as a capture ligand, that can, once bound, flag such cell, protein or virus as not-self and, therefore, facilitate removal of that cell, protein or virus from the organism. The nanoparticle can include an immuno stimulatory agent that stimulates an immune system component of interest to collect, aggregate, or migrate toward or around the nanoparticle, thereby facilitating binding of a capture ligand of the nanoparticle to bind with and flag the immune system cell as not-self and result in removal of the cell from the subject. As disclosed herein , the method of flagging self cells or proteins or viruses that are masked by non-recognized protein coats can treat a subject by facilitating the immune system to recognize the nanoparticle bound with the self agent as a now non-self agent, thereby facilitating removal of the agent from the subject.

### EXAMPLES

The following Examples have been included to provide guidance to one of ordinary skill in the art for practicing representative embodiments of the presently disclosed subject matter. In light of the present disclosure and the general level of skill in the art, those of skill can appreciate that the following Examples are intended to be exemplary only and that numerous changes, modifications, and alterations can be employed.

### Example 1. Particle Fabrication and Analytical Methods

Protein modification: The proteins were modified with either [succinimidyl 2-(biotinamido)-ethyl-1,3'-dithiopropionate] (degradable disulfide biotin linker) or sulfosuccinimidyl-6-[biotinamido]hexanoate (non-degradable biotin linker) using the following standard procedures.

Wyoming H3 HA: (Protein sciences): A solution of Wyoming HA (120 µg/mL, 1.75 mL, 210 µg total protein) was treated with 14.2 µL of a 10 mM [succinimidyl 2-(biotinamido)-ethyl-1,3'-dithiopropionate] (6 mg dissolved in 1 mL of H₂O). The mixture was shaken for 30 minutes, and then purified by dialysis using a 10 MWCO γ-irradiated slide-a-lyzer cassette (Pierce). The sample was dialyzed against 150 mL of H₂O, which was exchanged 7 times at 30 minute intervals. The protein was then recovered from the dialysis cassette and used for particle modification.

Recombinant mouse IL-12: (eBiosciences): A solution of mouse IL-12 (400 µg/mL, 5 µL, 2 µg total protein) was treated with 0.3 µL of a 10 mM [succinimidyl 2-(biotinamido)-ethyl-1,3'-dithiopropionate] (6 mg dissolved in 1 mL of H₂O). The mixture was shaken for 30 minutes, and then purified by drop dialysis using a 0.025 µm nitrocellulose membrane (Millipore). The sample was placed onto of the membrane, which was floated on 50 mL of H₂O, and dialyzed for 30 minutes. The protein was then recovered from the dialysis membrane and diluted 4-fold with H₂O for particle modification.

PEG Particle Fabrication and Modification: Particles were fabricated using the PRINT process using Fluorocur molds with the desired size and shape cavities. The molds were filled with a monomer mixture containing 20 wt% 2-aminoethyl methacrylate hydrochloride, 79 wt% polyethylene glycol dimethacrylate (MW ∼1000), and 1% 1-hydroxylcyclohexyl phenyl ketone. The filled molds were laminated against a PET sheet, and then cured with exposure to UV light. After curing, the molds were removed from the PET sheet leaving the particles isolated on the PET sheet. The particles were collected into solution (sterile H₂O) from the PET sheet by scraping with a polyethylene blade. The particles suspension was pelleted with centrifugation (16K x g, 10 minutes), the supernatant removed, and then redispersed into 70:30 ethanol:water by sonication. This procedure was repeated three times, and the particles were redispersed at 10 mg/mL in 70% ethanol and stored at 4 °C.

PLGA Particle Fabrication: Cationic PLGA particles for surface adsorption of HA (FLUVIRIN®) (Novartis, 2008-2009 seasonal) were prepared using the PRINT process using a 5 wt% solution in DMF of a 95:5 mixture of PLGA (50:50, 0.3 i.v.) and poly(dimethylaminomethacrylate) (MW ∼20,000). This mixture of polymers was molded with a FLUOROCUR® (Liquidia Technologies, Inc., North Carolina) mold containing 80 nm x 80 nm x 360 nm cavities. The particles were purified via centrifugation, and resuspended into 0.1 wt% polyvinyl alcohol solution.

HA protein (FLUVIRIN®) was adsorbed on to the surface by first removing all salt from the protein via dialysis against H₂O using a 10K MWCO dialysis membrane. For a target 10 wt% dosing of protein on the surface of the particle, 10 µg of protein (100 µL) was added to 0.1 mg of particles suspended 200 µL 0.1 wt% polyvinyl alcohol solution. The suspension was shaken at 4°C for 15 minutes, and then the particles were pelleted via centrifugation (16,000 x g, 10 minutes). The supernatant was removed, and the residual protein content was analyzed using the BCA assay. The particles were then redispersed in 0.4 mL of 0.1 wt% polyvinyl alcohol solution, such that the protein concentration was 25 µg/mL. The resulting 80 nm x 80 nm x 360 nm PLGA vaccine particles are shown in FIG. 6.

Biotin-Avidin Chemistry: Particles were surface modified with either [succinimidyl 2-(biotinamido)-ethyl-1,3'-dithiopropionate] (degradable disulfide biotin linker) or sulfosuccinimidyl-6-[biotinamido]hexanoate (non-degradable biotin linker) using the following standard procedure. A suspension of 200 nm x 200 nm x 200 nm particles in 70% ethanol (1 mL, 10 mg/mL) were pelleted, and then resuspended in 1 mL anhydrous DMF, three times. A solution containing 10 mg of [succinimidyl 2-(biotinamido)-ethyl-1,3'-dithiopropionate] and 70 mg of triethylamine in 0.5 mL of DMF was added to the particle suspension, and the mixture was then placed on a shaker table for 1 hour. The mixture was then purified by repeated centrifugation and resuspension in 70% ethanol (3 x 1 mL), and suspended to 10 mg/mL in 70% ethanol.

Surface Functionalization: The particles were coated with proteins in the following two step process. A suspension of biotinylated particles (0.2 mL, 10 mg/mL in 70% ethanol) were pelleted and washed into either 1 wt%% mouse albumin solution or 0.1 wt% polyvinyl alcohol solution twice by centrifugation and resuspension. The particles were resuspend to their original volume in the aqueous vehicle, and then an equivalent volume of 3 mg/mL avidin solution was added. The mixture was then shaken for 15 minutes at room temperature. The particles suspension was then purified via centrifugation and resuspension into the aqueous vehicle three times. The avidin-coated particles were then resuspended at 10 mg/mL in vehicle.

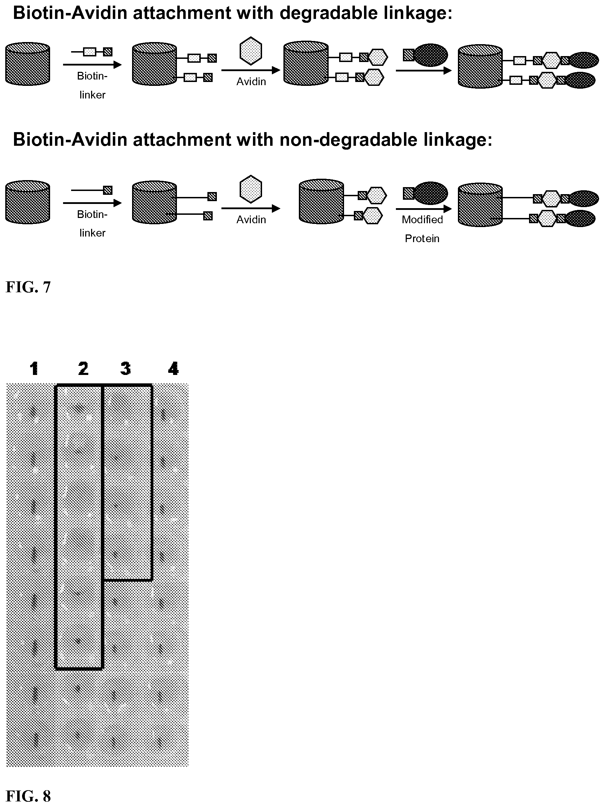

The particles were then coated with HA and/or IL-12 in a second step. The desired amount of protein was added to an eppendorf tube in H₂O followed by the addition of the avidin-coated particles. The suspension was shaken for 15 minutes at 4°C, and then pelleted by centrifugation. The supernatant was removed, and then particles were resuspended in vehicle to a volume, such that the desired amount of protein/injection was in 40 µL. If the particles were suspended in 0.1 wt% polyvinyl alcohol, the supernatants were analyzed for residual protein using the Bradford assay (typically >95% protein is removed from the supernatant). If the particles were suspended in 1% mouse albumin, the binding capacity of the particle was measured indirectly using the stoichiometry of binding a biotinylated protein to avidin on the particles. The avidin-coated particles were treated with a fluorescently labeled protein (bovine IgG labeled with both biotin and Hylite 647). The particles were pelleted, and the fluorescence intensity before and after particle treatment was compared and calibrated to a generated standard curve. The binding capacity was calculated for each particle, and then particles were dosed with HA and IL-12 at values equivalent or less than the measure binding capacity. A schematic of the biotin-avidin nanoparticles of the present invention are shown in FIG. 7.

Cationic Surface Adsorption: Unmodified particles (20% aminoethyl methacrylate HCL, 79% PEG_{1K}-dimethacrylate, 1% HCPK) were pelleted from ethanol suspension and washed into 0.1 wt% polyvinyl alcohol solution twice via centrifugation and resuspension. The particles (1 mg, 10 mg/mL) were added to a solution of HA (10 µg, 222 µL @ 45 µg/mL), which had been dialyzed against H₂O to remove NaCl from the protein preparation. The suspension was shaken at 4 °C for 15 minutes, and then the particle were pelleted and resuspended in 0.25 mL 0.1 wt% polyvinyl alcohol. The supernatant was analyzed for the presence of protein by the Bradford assay, and >95% of the protein was adsorbed to the particle. Recombinant mouse IL-12 can be adsorbed to particles in analogous manner alone or in combination with HA protein.

Analytical: HA ELISA A standard ELISA method was used to detect anti-HA antibody responses. In the assay, 96 well plates were coated with 50 ng / well of Hemagglutinin protein (H3 A / Wyoming/03/2003, Protein Sciences, Catalog Number 3006). Serum samples were tested in serial two-fold dilutions. Antibodies were detected using an anti-mouse IgG whole molecule with alkaline phosphatase developed in goat. (Sigma, catalog number=A3688) The assay was developed with Sigma Fast™ p-Nitrophenyl phosphate tablets (Sigma, Catalog number N2770-50SET) and read on a Molecular Devices SpectraMax M5 plate reader.

Antibody Staining for HA /IL12 coated particles: Particles with IL12 attached to the surface were exposed to a labeled antibody for IL12 (anti-mouse IL12 PE, BioLegend, Catalog number 505203) and washed. Particles appear red by fluorescence microscopy, due to the red labeled antibody that is specific to IL12.

Particles with HA attached to the surface were exposed to an antibody for HA (Anti-H3 Influenza Hemagglutinin Rabbit Polyclonal, Protein Sciences, catalog number 6100). The particles were washed leaving antibody bound to HA present on the particle surface. The antibody binding was recognized using an anti-rabbit secondary with a green label (anti-rabbit IgG AlexaFluor 488, Invitrogen, Catalog number A21206). Excess secondary was washed, and the particles were imaged by fluorescence microscopy to detect green fluorescence which would indicate the presence of HA on the particle surface.

Particles with IL12 and HA attached to the surface were exposed to a labeled antibody for IL12 (anti-mouse IL12 PE, BioLegend, Catalog number 505203) and to an antibody for HA (Anti-H3 Influenza Hemagglutinin Rabbit Polyclonal, Protein Sciences, catalog number 6100). The particles were washed leaving one antibody bound to HA present on the particle surface and the other antibody bound to IL12 on the surface. The HA antibody binding was recognized using an anti-rabbit secondary with a green label (anti-rabbit IgG AlexaFluor 488, Invitrogen, Catalog number A21206). Excess secondary was washed, and the particles were imaged by fluorescence microscopy to detect green fluorescence which would indicate the presence of HA on the particle surface and red fluorescence which would indicate the presence of IL12 on the particle surface, as shown in FIG. 8.

### Example 2: Immunogenicity of PRINT delivered Protein Vaccine Particles

Vaccine particles (composed of 79% Poly(ethylene glycol) dimethacrylate, 20% aminoethyl methacrylate HCl, 1 % HCPK, and then surface treated with degradable biotin linker) containing HA and IL12 on the surface were tested for their ability to stimulate Interferon gamma. First, spleenocytes were isolated from whole mouse spleens (BALB/c mice from Charles River Laboratories) and seeded into 96 well plates. Test particles were dosed on spleenocytes and allowed to incubate overnight. The supernatant from these cells was analyzed for the production of interferon gamma using a standard ELISA kit (Mouse INFg ELISA kit, eBiosciences, catalog number 88-7314-77). The data referenced Table 1. shows that IL12 is on particles and remains functional.

**Table 1.**

| **Sample** | **IFN-γ ELISA (IL-12 activity) Results (pg/ml)** |
|---|---|
| 200 nm Cationic PRINT particle with 1 µg of HA | NEG |
| 200 nm Cationic PRINT particle with 1 µg HA and 0.2 g IL12 | 233.90 |
| 1 µg of Fluvirin HA | NEG |
| 1 µg of Fluvirin HA and 0.2 µg of IL12 | 587.03 |

The in vivo study was designed to measure immune responses generated to the Influenza HA protein when delivered as a soluble protein or attached to vaccine particles with and without an immunostimulatory agent, IL-12. A cross-linked polyethylene glycol (PEG) based PRINT™ particle (composed of 79% Poly(ethylene glycol) dimethacrylate, 20% aminoethyl methacrylate HCl, 1 % HCPK, and then surface treated with degradable biotin linker) was used to present the HA antigen and the IL-12 protein. HA: IL12 is 100:1.

In vivo studies used female BALB/c mice from Charles River Laboratories. Mice were 7 weeks of age at the initiation of the studies. The animals' weight was between 15-25 g per mouse. In some groups, animals were given an intra-muscular (IM) dose of 40 uL (20 uL per flank) of particle solution so that each animal received 2 ug of HA protein on 0.150 mg of particles. In other groups, animals were given a rear footpad injection of 40 uL (20 uL per footpad) of particle solution so that each animal received 2 ug of HA protein. The study utilized 3 animals per group receiving identical doses. Animals were given a prime injection on day 1 followed by a boost injection on day 21. Blood was collected on Days 0, 8, 21 by retro-orbital bleeds for all groups. Animals were euthanized by CO₂ asphyxiation and confirmed by exsanguination on Day 29. Serum samples were collected from each of the bleeds and antibody titer was determined by ELISA.

### Example 3. Size and Shape Differences in Immunogenicity

This study was designed to evaluate immune responses generated to the Influenza HA protein when delivered as a soluble protein or attached to vaccine particles of multiple sizes and shapes. A cross-linked polyethylene glycol (PEG) based (composed of 79% Poly(ethylene glycol) dimethacrylate, 20% aminoethyl methacrylate HCl, 1 % HCPK, and then surface treated with a biotin linker surface modification) PRINT particle system will be used to present the HA antigen.

In vivo studies used female BALB/c mice from Charles River Laboratories. Mice were 7 weeks of age at the initiation of the studies. The animals' weight was between 15-25 g per mouse. Animals were given an intra-muscular (IM) dose of 40 uL (20 uL per flank) of particle solution so that each animal received 2 ug of HA protein with a particle dose ranging between 0.08 and 0.3 mg of particles/injection depending on binding capacity. This study utilized 6 animals per group of a given test sample. Animals were given a prime injection on day 1 followed by a boost injection on day 21. Blood was collected on Days 0 and day 20 by retro-orbital bleeds for all groups. Animals were euthanized by CO₂ asphyxiation and confirmed by exsanguination on Day 29. The necropsy will include blood and spleen collection. Serum samples were collected from each of the bleeds and antibody titer was determined by ELISA. The results are shown in FIGS. 2 and 3.

### Example 4. Degradable Versus non-degradable linker:

This study was designed to evaluate immune responses generated to the Influenza HA protein when delivered as a soluble protein or attached to vaccine particles using different linker group chemistries. A cross-linked polyethylene glycol (PEG) (composed of 79% Poly(ethylene glycol) dimethacrylate, 20% aminoethyl methacrylate HCl, 1 % HCPK, and then surface treated with degradable biotin linker) based PRINT particle system will be used to present the HA antigen.

In vivo studies used female BALB/c mice from Charles River Laboratories. Mice were 7 weeks of age at the initiation of the studies. The animals' weight was between 15-25 g per mouse. Animals were given an intra-muscular (IM) dose of 40 uL (20 uL per flank) of particle solution so that each animal received 2 ug of HA protein on between 0.1 and 0.3 mg of particles depending on particle binding capacity. This study utilized 6 animals per group of a given test sample. Animals were given a prime injection on day 1 followed by a boost injection on day 21. Blood was collected on Days 0 and day 20 by retro-orbital bleeds for all groups. Animals were euthanized by CO₂ asphyxiation and confirmed by exsanguination on Day 29. The necropsy will include blood and spleen collection. Serum samples were collected from each of the bleeds and antibody titer was determined by ELISA. The results are shown in FIGS. 4 and 5.

## Claims

1. A vaccine composition for administration to a subject comprising:
a plurality of particles, wherein each particle:
is a nanometer-sized particle,
is coupled with an antigen, and
is fabricated with an aspect ratio of from 2:1 to 4:1,
wherein the vaccine composition is engineered to trigger immunogenicity when administered to the subject, wherein the immunogenicity decreases with increased aspect ratio of said particle.

2. The vaccine composition of claim 1, wherein the aspect ratio is 3:1.

3. The vaccine composition of claim 1 or 2, wherein the particles are further coupled with an immunostimulating agent.

4. The vaccine composition of claim 3, wherein at least one of the immunostimulating agent and the antigen is coupled with the particles by a non-degradable linker, or a degradable linker.

5. The vaccine composition of claim 3 or 4, wherein the immunostimulating agent is physically separated from the antigen.

6. The vaccine composition of any of claim 3 to 5, wherein the particles are configured to time release different doses of the antigen and the immunostimulating agent.

7. The vaccine composition of claim 1 or 2, wherein the particles are configured to time release different doses of the antigen.

8. The vaccine composition of any of claim 1 to 7, wherein the particles are configured to degrade at predetermined rates and/or in response to predetermined environmental conditions.

9. The vaccine composition of any of claim 1 to 8, wherein the particles are configured to provide both priming and boosting capability in a dose of the vaccine composition.

10. A vaccine composition of claim 1 wherein:
the plurality of particles are substantially equivalently sized and shaped and comprise a PLGA composition; and
the antigen, preferably HA protein, is adsorbed on a surface of the particles.

11. The vaccine composition of claim10, wherein substantially each particle of the plurality of particles has a first dimension of 80 nanometers.

12. The vaccine composition of claim 1, wherein the composition comprises biodegradable materials.

## Patentansprüche

1. Impfstoffzusammensetzung zur Verabreichung an ein Individuum, die umfasst:
eine Vielzahl von Partikeln, wobei jeder Partikel:
ein Nanometer-großer Partikel ist,
mit einem Antigen gekoppelt ist, und
hergestellt ist mit einem Seitenverhältnis (Aspect Ratio) von 2:1 bis 4:1,
wobei die Impfstoffzusammensetzung so konstruiert ist, um Immunogenität bei Verabreichung an ein Individuum auszulösen, wobei die Immunogenität mit zunehmendem Seitenverhältnis des Partikels abnimmt.

2. Impfstoffzusammensetzung nach Anspruch 1, wobei das Seitenverhältnis 3:1 ist.

3. Impfstoffzusammensetzung nach Anspruch 1 oder 2, wobei die Partikel zudem mit einem immunstimulierenden Agens gekoppelt sind.

4. Impfstoffzusammensetzung nach Anspruch 3, wobei mindestens eines des immunstimulierenden Agens und des Antigens mit den Partikeln durch einen nicht-abbaubaren Linker oder einen abbaubaren Linker gekoppelt ist.

5. Impfstoffzusammensetzung nach Anspruch 3 oder 4, wobei das immunstimulierende Agens physisch vom Antigen getrennt ist.

6. Impfstoffzusammensetzung nach einem der Ansprüche 3 bis 5, wobei die Partikel so gestaltet sind, dass zeitlich festgelegt verschiedene Dosen des Antigens und des immunstimulierenden Agens freigesetzt werden.

7. Impfstoffzusammensetzung nach Anspruch 1 oder 2, wobei die Partikel so gestaltet sind, dass zeitlich festgelegt verschiedene Dosen des Antigens freigesetzt werden.

8. Impfstoffzusammensetzung nach einem der Ansprüche 1 bis 7, wobei die Partikel so gestaltet sind, dass sie zu vorbestimmten Raten und/oder als Reaktion auf vorbestimmte Umweltbedingungen abgebaut werden.

9. Impfstoffzusammensetzung nach einem der Ansprüche 1 bis 8, wobei die Partikel so gestaltet sind, sowohl die Fähigkeit zum Priming als auch zum Boosting in einer Dosis der Impfstoffzusammensetzung bereitzustellen.

10. Impfstoffzusammensetzung nach Anspruch 1, wobei:
die Vielzahl der Partikel im Wesentlichen von gleicher Größe und Form sind und eine PLGA Zusammensetzung umfassen; und
das Antigen, bevorzugt ein HA Protein, an einer Oberfläche der Partikel adsorbiert ist.

11. Impfstoffzusammensetzung nach Anspruch 10, wobei im Wesentlichen jeder Partikel der Vielzahl der Partikel eine erste Dimension von 80 Nanometern hat.

12. Impfstoffzusammensetzung nach Anspruch 1, wobei die Zusammensetzung biologisch abbaubare Materialien umfasst.

## Revendications

1. Composition vaccinale destinée à être administrée à un sujet comprenant :
une pluralité de particules, dans laquelle chaque particule :
est une particule ayant une taille de l'ordre du nanomètre,
est couplée à un antigène, et
est fabriquée avec rapport d'aspect compris entre 2:1 et 4:1,
où la composition vaccinale est manipulée pour provoquer une immunogénicité lorsqu'elle est administrée au sujet, où l'immunogénicité diminue avec l'augmentation du rapport d'aspect de ladite particule.

2. Composition vaccinale selon la revendication 1, dans laquelle le rapport d'aspect est de 3:1.

3. Composition vaccinale selon la revendication 1 ou 2, dans laquelle les particules sont en outre couplées à un agent immunostimulateur.

4. Composition vaccinale selon la revendication 3, dans laquelle au moins un de l'agent immunostimulateur et de l'antigène est couplé aux particules par un segment de liaison non dégradable, ou un segment de liaison dégradable.

5. Composition vaccinale selon la revendication 3 ou 4, dans laquelle l'agent immunostimulateur est physiquement séparé de l'antigène.

6. Composition vaccinale selon l'une quelconque de la revendication 3 à 5, dans laquelle les particules sont configurées pour libérer différentes doses de l'antigène et de l'agent immunostimulateur au cours du temps.

7. Composition vaccinale selon la revendication 1 ou 2, dans laquelle les particules sont configurées pour libérer différentes doses de l'antigène au cours du temps.

8. Composition vaccinale selon l'une quelconque de la revendication 1 à 7, dans laquelle les particules sont configurées pour se dégrader à des taux prédéterminés et/ou en réponse à des conditions environnementales prédéterminées.

9. Composition vaccinale selon l'une quelconque de la revendication 1 à 8, dans laquelle les particules sont configurées pour conférer à la fois une capacité de sensibilisation et de rappel dans une dose de la composition vaccinale.

10. Composition vaccinale selon la revendication 1, dans laquelle :
la pluralité de particules ont une taille et une forme essentiellement équivalentes et comprennent une composition de PLGA ; et
l'antigène, de préférence une protéine HA, est adsorbé sur une surface des particules.

11. Composition vaccinale selon la revendication 10, dans laquelle essentiellement chaque particule de la pluralité de particules possède une première dimension de 80 nanomètres.

12. Composition vaccinale selon la revendication 1, où la composition comprend des matériaux biodégradables.
